# EUROPEAN PATENT SPECIFICATION

(11) **EP 0 901 494 B1**
(45) Date of publication and mention of the grant of the patent: **04.12.2002**
(21) Application number: 96931487.1
(22) Date of filing: 06.09.1996
(51) Int. Cl.: C07D 498/10, G03C 1/685

(54) **PHOTOCHROMIC SPIROXAZINES WITH AN ASYMMETRIC MONOCYCLIC SUBSTITUENT, COMPOSITIONS AND ARTICLES CONTAINING THEM**
PHOTOCHROME SPIROXAZINE MIT EINEM ASYMMETRISCHEN MONOZYLKLISCHEN SUBSTITUENTEN, ZUSAMMENSETZUNGEN UND PRODUKTE DIE DIESE ENTHALTEN
SPIROXAZINES PHOTOCHROMIQUES AVEC A SUBSTITUANT ASYMETRIQUE MONOCYCLIQUE, COMPOSITIONS ET PRODUITS LES RENFERMANT

(30) Priority: 11.09.1995 FR 9510618; 16.01.1996 US 609937
(43) Date of publication of application: 17.03.1999
(73) Proprietor: CORNING INCORPORATED, Corning, N.Y. 14831 (US)
(72) Inventor: CHAN, You-Ping, F-69008 Lyon (FR)
(74) Representative: Le Roux, Martine
(86) International application number: US9614377
(87) International publication number: WO97010241

(56) References cited:
- DE-A- 1 927 849
- US-A- 5 139 707
- CHEMICAL ABSTRACTS, 04 September 1989, Volume 111, number 10, Abstract Number 111:87504z, NISSAN MOTOR CO. LTD., "Spirooxazine Photochromic derivative and Photosensitive Material Using Same".
- CHEMICAL ABSTRACTS, Volume 114, Number 14, 08 April 1991, MITSUBISHI KASEI CORP., "Spiroindolinoquinoxazine Compounds and Photosensitive Materials Containing Them", Abstract Number 114:133085a.

## Description

The present invention concerns novel compounds of the spiroxazine type presenting, in particular, photochromic properties. It also concerns the photochromic compositions and ophthalmic articles (e.g., lenses) which contain these spiroxazines.

The photochromic compounds are capable of changing color due to the influence of a poly- or monochromatic light (e.g., UV) and they are capable of covering their initial content, when the irradiation with light stops, or due to the influence of a poly- or monochromatic light which is different from the first light, or due to the influence of the temperature and/or of a poly- or monochromatic light which is different from the first one.

These photochromic compounds are applied in various fields, for example, for the manufacture of ophthalmic lenses, contact lenses, some sunshades, filters, optics for cameras or photography apparatuses or other optical devices or observation devices, glass partitions, decorative objects, elements of displays or for the storage of information by optical inscription (encoding).

In the field of ophthalmic optics, and in particular in the field of eyeglasses, a photochromic lens, comprising one or more photochromic compounds, must present:
- a high transmission in darkness or in the absence of sunlight,
- a low transmission (high colorability) when exposed to irradiation by sunlight,
- an appropriate kinetics of coloration and decoloration,
- a tint which is acceptable to the consumer (gray or chestnut brown, preferably), with, preferably, maintenance of the selected tint during the course of the coloration and the decoloration of the lens,
- a maintenance of the performances of the characteristics in a temperature range of 0-40°C,
- a high durability, because the objectives intended are sophisticated and, therefore expensive, corrective lenses.

These lens characteristics are in fact determined by the active photochromic compounds which, in addition, must be perfectly compatible with the organic or mineral support which constitutes the lens.

Moreover, it should be noted that the obtention of a gray or chestnut brown tint may require the use of at least two photochromes of different colors, that is having distinct maximum absorption wavelengths in the visible range (λₘₐₓ). This association thus imposes still other requirements on photochromic compounds. In particular, the kinetics of coloration and decoloration of the two or more associated active photochromic compounds must be essentially identical. The same applies to their stability over time and, also, to their compatibility with a plastic or mineral support.

Among the numerous photochromic compounds described in the prior art, one can cite the indolinospironaphtoxazines described in U.S. Patent Nos. 3,578,602, 3,562,172, 4,215,010, 5,139,707; European Patent Nos. 0,171,909, 0,313,941; French Patent No. 2,647,789; European Patent No. 0,600,669:

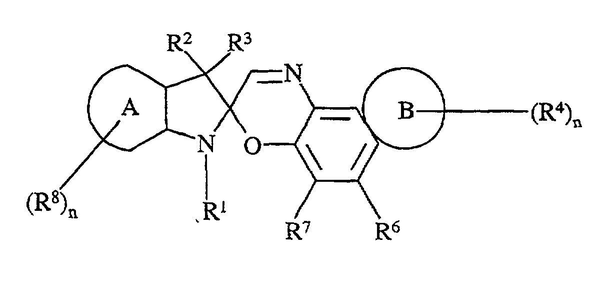

The group R¹ of these molecules represent linear or branched alkyls, alkylaryls or alicyclics. These compounds are considered to meet the specifications defined above.

In fact, while these compounds have indeed one or more of the wanted basic properties, such as high transmission in darkness, high colorability when exposed to sun radiation, absorption in the blue or violet [spectrum] (570-630 nm), rapid kinetics of coloration and decoloration, all the compounds described to this day do not have the complete combination of the desired properties that are required for the production of satisfactory articles, which can be manufactured industrially.

Whereas the prior art teaches how to modify the absorption band by the addition of substituents to the different positions of the rings and also how to modify the kinetics of decoloration, in contrast, it does not teach how to increase the colorability of these molecules without increasing the residual coloration in the inactivated state and, above all, on how to make them photochemically stable so as to allow their use on an industrial scale. Indeed, without a high stability, these expensive molecules, introduced into a sophisticated lens, cannot be used.

It is the merit of the applicant to have found unexpectedly that the presence of an asymmetric monocyclic substituent group on the nitrogen atom of the indoline or portion of certain indolinospiroxazines allows the solution of the problem of the residual coloration and of the colorability, which is essential for the indicated applications.

The originality of the invention resides in the surprising effect of the asymmetric monocyclic group which increases the colorability of the spiroxazines, without increasing their residual coloration, while ensuring a good photostability.

Thus, the present invention concerns a compound, in particular, a photochromic compound, having the following general formula (I): 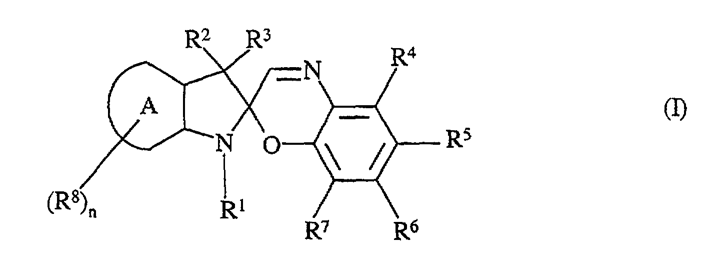 in which,
- R¹ is an asymmetric aliphatic monocyclic group which is directly connected to the nitrogen atom, having from 5 to 7 members, and possibly comprising at least one heteroatom, and having the formula 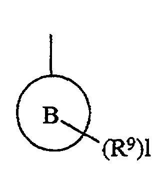 where 1 = 1-12, R⁹ represents H or an alkyl, alkoxy, diallcylamino, alkyl ester or CN group, provided that at least one of the R⁹ is not H.
- R², R³ are identical or different and they represent an alkyl group, linear or branched, of 1-12 carbon atoms, an alkenyl, alkynyl, aryl, alkylaryl, cycloalkyl; or R² and R³ can, optionally, combine to form a carbocyclic or heterocyclic group having 5 to 10 atoms,
- R⁴, R⁵, R⁶ and R⁷ are identical or different and they represent:
   ■ hydrogen,
   ■ an alkyl, cycloalkyl, alkenyl, alkynyl, aryl (preferably phenyl, naphthyl mono-, di- or trisubstituted by electron donor or acceptor groups), heteroaryl, aryloxy or aralkyl, said group being optionally halogenated,
   ■ a halogen, preferably F, Br, Cl,
   ■ OR, SR, -OCOR, or -COOR, with R = H, alkyl and/or cycloalkyl and/or aryl,
   ■ a (poly)ether, a (poly)amide, a (poly)carbonate, a (poly)carbamate, a (poly)urea or a (poly)ester,
   ■ an amino radical which gives rise, once it is bound in (I), to a primary, secondary or tertiary amine, said amine being alkyl, aryl or aralkyl, mono- or disubstituted depending on its nature,
      or an aminocyclic radical containing one or more heteroatoms,
   ■ or an electron withdrawing group selected, preferably, from the group comprising CF₃, CN, NO₂, SCN,
- where at least two of the radicals R⁴, R⁵, R⁶, R⁷, preferably borne by two adjacent carbon atoms, can combine to form at least one aromatic ring having 5 or 6 members or aliphatic ring having 5 to 7 members, preferably 5 or 6 members, said ring(s) comprising, optionally, at least one heteroatom, so as to form at least one heterocyclic ring, the latter being optionally substituted by one or more radicals, which may be identical or different, and have the same definition as given above for R⁴ to R⁷,
- A represents a (hetero)aromatic ring (containing, for example, at least one nitrogen atom, which is possibly substituted by one or more radicals R⁸, which may be identical or different and have the same definition as given above for R⁴ to R⁷, except hydrogen,
- n is a whole number and when n ≥ 2, two of the radicals R⁸ can possibly combine to form at least one aromatic or heteroaromatic ring.

The term "asymmetric" in the expression "asymmetric aliphatic monocyclic group" signifies that the plane which is perpendicular to the ring B of group R¹ and including the segment of a straight line representing the bond between the ring B and the nitrogen atom is not a plane of symmetry for the group R¹, with the understanding that this reasoning is based on the formula developed in a plane of the group R¹.

Specific examples of groups R¹ are the following: 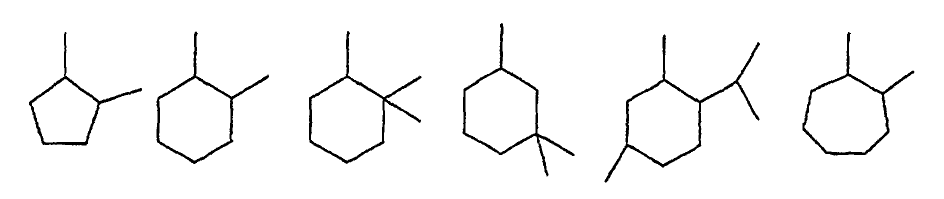 among many others.

Preferred compounds of the invention have the following formula (I'): 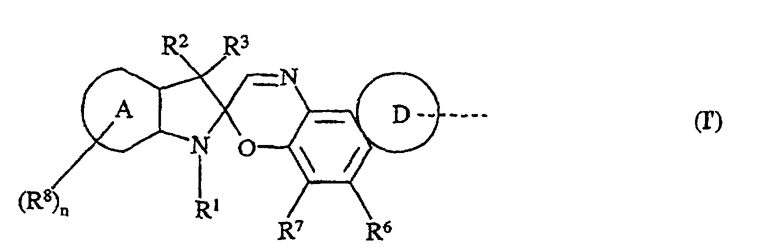 where A, R¹ to R³, R⁶ to R⁸ and n are as defined above, and D is an aromatic or aliphatic ring with 5 to 7 members, optionally comprising a heteroatom, which may or may not be substituted by one or more radicals, which may be identical or different, and have the same definition as given for R⁴ to R⁷, except hydrogen.

According to a particularly preferred embodiment of the invention, the ring A is a phenyl group and R⁴ and R⁵ combine to form an aromatic ring. This corresponds to the following formula (I"): 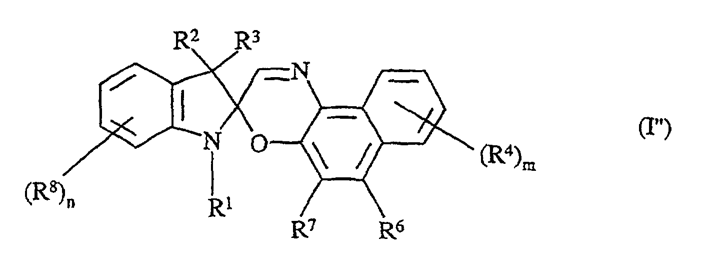 in which R¹ to R⁴ and R⁶ to R⁸ are as defined above, and n assumes the values 0 to 4, while m assumes the values 1 to 4.

Among the substituents which can be considered for the compounds with Formulas (I), (I') and (I") according to the invention, groups R⁴ to R⁹ must be considered which comprise and/or form at least one reactive function for polymerization and/or crosslinking, selected, preferably, from the following list: alkenyl--preferably vinyl--methacryloyl, acryloyl, acryloxyalkyl, methacryloxyalkyl or epoxy.

Thus, the photochromic compounds according to the invention can be designed as monomers, of different types or not, which can react between themselves or with other comonomers, to form homopolymers and/or copolymers, which bear a photochromic group and which have the mechanical properties of macromolecules. It follows that one of the objects of the present invention consists of these homopolymers or copolymers comprising (co)monomers and/or of crosslinkages, consisting at least in part of photochromic compounds (I), (I') and (I") according to the invention.

In the same order of ideas, the above-mentioned compounds (I), (I') and (I") can be considered to be crosslinking agents which have reactive functions capable of allowing the formation of bridges between polymer chains which may or may not be of photochromic type. The crosslinkages, which can thus be obtained, also constitute another object of the present invention.

In general, in the preceding formulas, the following designations are used, according to the invention:
- "alkyl", referring preferably to a linear or branched hydrocarbon group having from 1 to 12 carbon atoms,
- "alkoxyl", referring to a group of O-alkyl type preferably having from 1 to 10 carbon atoms,
- "aryl", referring to an aromatic hydrocarbon group containing at least 6 carbon atoms,
- "heteroaryl", referring to an aromatic hydrocarbon group comprising of at least 5 atoms, of which at least one is a heteroatom,
- "aralkyl", a group comprising of at least one alkyl and at least one aryl, as defined above,
- "heteroatom", atoms different from C and H, belonging preferably to the following group: N, O, and S.

The photochromic compounds used which are particularly preferred in the context of the invention are thus, as can be concluded from the above, indolinospironaphthoxazines or indolinospirobenzoxazines.

The most advantageous indolinospiroxazines include those having the formula: 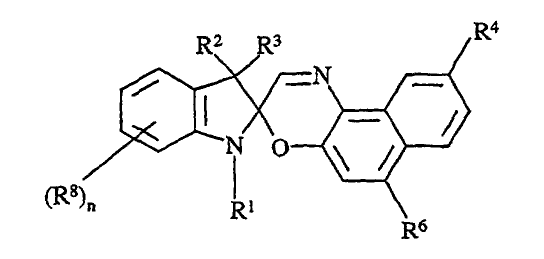 where R¹ = 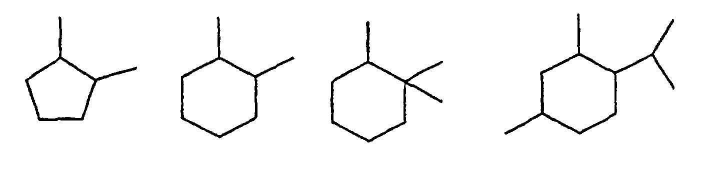
n = 0, 1 or 2 and
R², R³ = C₁-C₅ alkyl,
R⁴ = H, OMe,
R⁶ = H, OMe or amino,
R⁸ = Me, OMe or CF₃.

It is the merit of the applicant to have disclosed these compounds, because they present particularly advantageous photochromic properties. More specifically, they have high colorability, particularly in the blue region of the spectrum. They are thus well suited to combination--observing compatibility and complementarity requirements--with photochromes which absorb in the yellow, orange, red and violet spectra, so as to obtain a broad coverage of the visible absorbance spectrum and thus coloration tints which are just chestnut brown or dark gray.

The sensitivity, as well as the height and the area of their λₘₐₓ peaks in the visible range reach satisfactory values.

These compounds are also perfectly stable and compatible with support matrices made of organic polymers or of mineral material, both in a form included in the matrix and in the form of a coating.

In solution, or in a polymer matrix, the compounds according to the invention are colorless or slightly colored in the initial state and rapidly develop an intense coloration under UV light (365 nm) or a light source of the sun radiation type. Finally, they quickly restore their initial color when the irradiation stops.

The compounds of the invention can be obtained by the condensation of an indoline derivative substituted with an asymmetric aliphatic monocyclic group R¹ and an aromatic nitroso alcohol derivative such as those described, for example, in U.S. Patent Nos. 3,578,602, 4,634,767, 4,913,544 and European Patent No. A 600,669. This reaction can take place in solvents such as ethanol, toluene or dichloroethane.

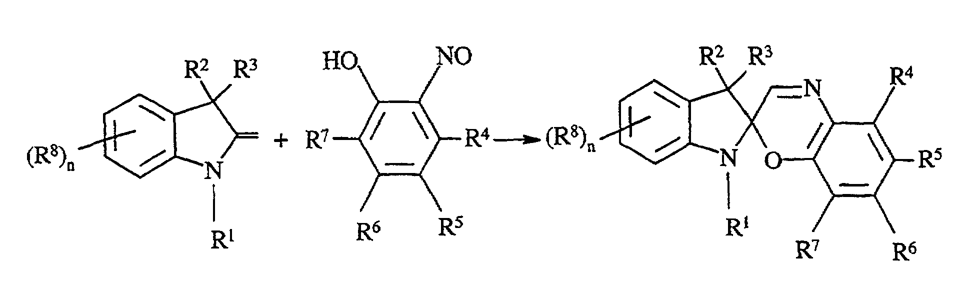

The indoline derivatives are obtained by methods which are adapted from the literature.

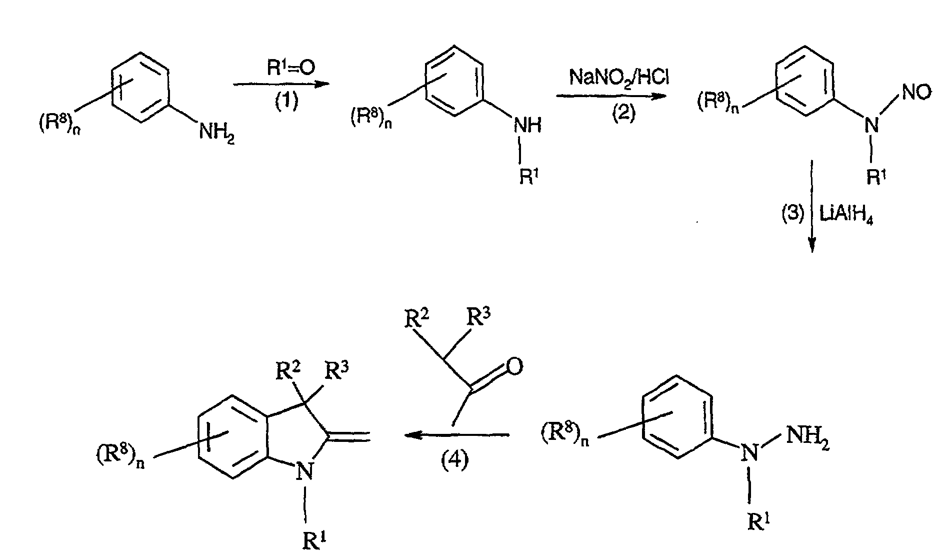

Step 1 is performed according to a procedure described in Katritzky et al., Tetrahedron, 1991, Vol. 47, p. 2683. The nitrosation of the amine (step 2) is conducted by a reaction with sodium nitrite-hydrochloric acid and the reduction of the nitroso derivative (step 3) is conducted by the reaction of LiAlH₄ in THF (Fridman et al., Russian Chemical Reviews, 1971, 40(1), 34). The last step of the synthesis (4) is conducted by reacting hydrazine with the appropriate ketone in an acidic medium, for example, hydrochloric acid/ethanol or acetic acid (for a general review of this reaction one can consult Robinson "Fischer indole synthesis", Wiley-Interscience, 1982).

In the case of applications of compounds according to the present invention, it should be noted that they can be used as a photochromic material which is dispersed in the superficial part or in the composition of a polymer or mineral matrix. They can also be used in solution.

A photochromic solution can be obtained by dissolving the compound in an organic solvent, such as toluene, dichloromethane, tetrahydrofuran or ethanol. The solutions obtained are in general colorless and transparent. When exposed to sunlight, they developed a strong coloration and their colorless state is restored when they are placed in a zone with less exposure to solar radiation or, in other words, when they are no longer exposed to UV radiation. In general, it is sufficient to use a very small concentration of product (on the order of 0.01-5%) to obtain an intense coloration.

The most intense applications are those in which the photochrome is dispersed uniformly within or on the surface of a polymer, copolymer or mixture of polymers. A great variety of methods of implementation can be considered. Those known to persons skilled in the art include, for example, diffusion in the (co)polymer, from a suspension or solution of the photochrome, in a silicone oil, in an aliphatic or aromatic hydrocarbon, in a glycol, or from another polymer matrix. The diffusion is routinely conducted at a temperature of 50-200°C for a duration of 15 min to several hours, depending on the nature of the polymer matrix.

Another implementation technique consists in mixing the photochrome in a formulation of polymerizable substances, in depositing this mixture on a surface or in a mold and then conducting the polymerization.

These implementation techniques and others are described in the article by CRANO et al. "Spiroxazines and their use in photochromic lenses" published by Applied Photochromic Polymer Systems, Published by Blackie and Son Ltd-1992.

According to a variant of the invention, it is also possible to consider grafting the photochromes onto (co)polymers. Thus, another object of the invention consists of (co)polymers to which at least one of the photochromes described above has been grafted.

Examples of preferred polymer materials for the optical applications of the photochromic compounds according to the invention include the following products:
- polyacrylate or polymethacrylate of alkyl, of cycloalkyl, of aryl or of arylalkyl (mono, di, tri or tetra), optionally halogenated or comprising of at least one ether and/or ester and/or carbonate and/or carbamate and/or thiocarbamate and/or urea and/or amide group(s),
- polystyrene, polycarbonate (e.g., bisphenol-A polycarbonate, allyl polycarbonate diethylene glycol, polyepoxy, polyurethane, polythiourethane, polysiloxane, polyacrylonitrile, polyamide, aliphatic or aromatic polyester, vinyl polymers, cellulose acetate, cellulose triacetate, cellulose acetate propionate or polyvinylbutyral,
- copolymers of two or several types of monomer or mixtures of polymers mentioned above, preferably polycarbonate-polyueethane, poly(meth)acrylate-polyurethane, polystyrene-poly(meth)acrylate or polystyrene-polyacrylonitrile, preferably a mixture of polyester or of polycarbonate or of poly(meth)acrylate.

The quantity of photochrome used depends on the desired degree of darkening. Usually a quantity of 0.001-20 wt% is used.

The photochromic compounds according to the invention can be used alone or in a mixture with other products to form a composition which can be in solid or liquid form, for example, in a solution or in a dispersion, as already indicated above. These compositions, which constitute another object of the invention, can thus comprise the compounds (I), (I') and (I") of the invention and other complementary photochromic compounds which allow the obtention of dark colorations, for example, gray or brown, which are desired by the public in applications such as ophthalmic or sun protection eyewear. These complementary photochromic compounds have an λₘₐₓ and a absorbance spectrum in visible such that after association with the compounds of the invention, impart the desired tint to the mixture of the activated photochromes.

The photochrome(s), which can be associated with the compounds of the invention, islare those known to a person skilled in the art and described in the literature, for example, chromenes (U.S. Patent Nos. 3,567,605, 5,238,981; World Patent No. 9,422,850; European Patent No. 0,562,915), spiropyranes or naphthospyropanes [sic] (U.S. Patent No. 5,238,981) and spiroxazines (J.C. CRANO et al., "Applied Photochromic Polymer Systems", Publisher Blackie & Son Ltd., 1992, Chapter 2).

These compositions according to the invention can also comprise:
- nonphotochromic dyes which allow an adjustment of the tint,
- and/or one or more stabilizers, such as, for example, an antioxidant,
- and/or one or more anti-UV agents,
- and/or one or more antiradical compounds,
- and/or one or more deactivators of photochemical excitation states.

These additives can allow an improvement of the durability of said compositions.

According to another of its aspects pertaining to the application of the photochromic compounds (I), (I') and (I"), the present invention also relates to ophthalmic articles, such as articles of ophthalmic or sun protection eyewear, comprising at least one compound according to the invention and/or at least one (co)polymer formed, at least in part, of recurrent units of type (I), (I') or (I") and/or at least one composition comprising the compounds (1), (I') or (I") according to the invention, as defined above, and/or at least one matrix, as defined above, made of an organic polymer material or of a mineral material or of a mineral-organic hybrid material incorporating at least a compound of the invention.

In practice, the articles which are most referred to by the present invention are photochromic ophthalmic or sunprotection lenses, window panes (windows for buildings, for locomotives, automobiles), the optical devices, the decorative articles, the articles for sun protection, the storage of data, etc.

The present invention will be understood better in light of the following examples of photochromic synthesis and validation of compounds (I), (I') and (I") which it concerns.

### Examples

### Synthesis and properties of photochromic compounds (1)-(4) according to the invention (Examples 1-4)

The formulae of compounds (1)-(4) are given below (see Table I).

### Example 1: Synthesis of compound (1)

### Step 1:

### Synthesis of 2-methylcyclohexylphenylamine

In a 100-mL flask, the following mixture is heated at reflux for 30 h: 9.3 g aniline, 11.2 g 2-methylcyclohexanone and 12 g benzotriazole. Then, 200 mL of methanol and 5 g of NaBH₄ in portions are added to the mixture, which is then heated at reflux for 30 min. The solution is allowed to cool, and 100 mL of water are added to the mixture, which is extracted with 2 x 100 mL diisopropyl ether. The organic phase is recovered, dried over magnesium sulfate and reduced to dryness. In this manner, 7 g of the desired amine are produced.

### Step 2:

### Synthesis of 1-(2-methylcyclohexyl)-1-phenylhydrazine

The amine from the preceding step (7 g) is suspended in 100 mL of hydrochloric acid (1N), and then the mixture is maintained at 0°C with stirring. An aqueous solution of NaNO₂ (2 g in 20 mL of water) is then added to the mixture in small portions. The temperature is then allowed to rise to the ambient temperature, and the nitroso derivative is extracted with 2 x 100 mL diisopropyl ether. After evaporation of the solvent, 8 g of product are recovered. This product is then added slowly and in small portions into tetrahydrofuran (80 mL) containing 3 g LiAlH₄ and, then, the mixture is heated at reflux for 1 h. The mixture is subsequently cooled to 0°C, and then the excess of hydride is neutralized with an aqueous soda solution. Next, 30 g Na₂SO₄ are added, and the organic phase is recovered by filtration and reduced to dryness. In this manner 7 g of the desired hydrazine are produced.

### Step 3:

### Synthesis of the 2-methylene indoline derivative

In a 100-mL flask, 7 g hydrazine from the preceding step and 3 g 3-methyl-2-butanone in 30 mL ethanol containing 1 drop of acetic acid at 50°C are reacted. Then 5 mL concentrated hydrochloric acid are added, and the mixture is heated at reflux temperature for 10 min. The medium is then neutralized with soda and the indole derivative is extracted with 100 mL diisopropyl ether. After evaporation of the solvent, 3 g of the desired product are obtained.

### Step 4:

### Synthesis of spiroxazine (1)

The product of the preceding step (3 g) and 1.8 g 1-nitroso-2-naphthol are dissolved in 30 mL ethanol, and then the mixture is heated at 70°C for 30 min. The solvent is then evaporated under a vacuum, and the product is then isolated by chromatography on a silica column with toluene as eluant. The product obtained (1 g) is crystallized in heptane. 450 mg of a slightly green product are isolated. Its structure is confirmed by NMR spectroscopy.

### Example 2: Synthesis of compound (2)

### Step 1:

In a 100-mL flask, the following mixture is heated at 120°C for 24 h: 12 g 3,4-dimethylaniline, 11 g 2-methylcyclohexanone and 12 g benzotriazole. The 2-methylcyclohexyl-2,4-dimethylphenylamine is isolated after reduction, as in step 1 of Example 1. 18 g of an oily product are isolated.

### Steps 2, 3 and 4:

These steps are conducted as before. The spiroxazine (2) is synthesized from 3.5 g of the indoline derivative and 2.2 g 1-nitroso-2-naphthol in ethanol at 60°C for 2 h. 1.5 g of product are isolated after chromatography. Its structure is confirmed by NMR spectroscopy. The latter shows the existence of several isomers, because of the position of the two methyls on the phenyl of the indole (4,5-dimethyl and 5,6-dimethyl derivative) and because of the position of the nitrogen on the ring (cis and trans with respect to the 2-methyl group).

### Example 3: Synthesis of compound (3)

### Step 1:

In a 250-mL flask, equipped with a Dean-Stark separator, the following mixture is heated at reflux: 12 g 3,4-dimethylaniline, 20 g menthone, 14 g benzotriazole and 200 mL toluene. After recovery of the theoretical quantity of water (16 h), the mixture is reduced to dryness and then dissolved in 200 mL methanol, and the product is reduced with NaBH₄, as above. 25 g of an oily product are isolated.

### Steps 2, 3 and 4:

These steps were conducted as before, the spiroxazine (3) is synthesized from 5 g of the indoline derivative and 3.5 g 1-nitroso-2-naphthol in 60 mL. ethanol at 50°C for 1 h. 2 g of product are isolated after chromatography. Its structure is confirmed by NMR spectroscopy.

### Example 4: Synthesis of compound (4)

This spiroxazine (4) is synthesized from 6 g of the indoline derivative of Example 3 and 4 g 1-nitroso-7-hydroxy-2-naphthol in the ethanol at 60°C for 1 h. The intermediate product is then methylated with dimethyl sulfate in acetone in the presence of potassium carbonate. 2.2 g of product are isolated after chromatography. Its structure is confirmed by NMR spectroscopy.

### Applications

### Example 5: Incorporation of compounds (1) through (4) in a polyacrylate

General procedure: 10 mg of each one of compounds (1) through (4) are dissolved in tetraethoxylated bisphenol A dimethyl methacrylate, marketed under the name DIACRYL 121 by the company AKZO and also containing 40 mg of 2',2'-azobis(2-methylbutyronitrile). The solution is then degassed, rendered inert with argon, and then poured in a lens mold made of glass having a diameter of 8 cm and a thickness of 2 mm. The mold is then placed in an oven at 70°C for 12 h. After removal from the mold, a transparent and rigid lens is obtained. When exposed to solar-type radiation, the glass quickly develops an intense blue coloration and it again becomes colorless in darkness. The photochromic characteristics are given in Table I below. For comparison the characteristics of compounds C1, C2, C3, C4 and C5 of the prior art are also given in Table I below.

### Table I

### Legends:

- λₘₐₓ measured in D121 in a thickness of 2 mm following exposure to a xenon lamp, 60,000 lx, at 22°C,
- T0 = initial transmission (unactivated state) measured at λₘₐₓ,
- TD15 = transmission after 15 min of exposure measured at λₘₐₓ,
- IOD = induced optical density (Log(T0/TD15)),
- R5 = % of recovery of the initial transmission after 5 min of decoloration.

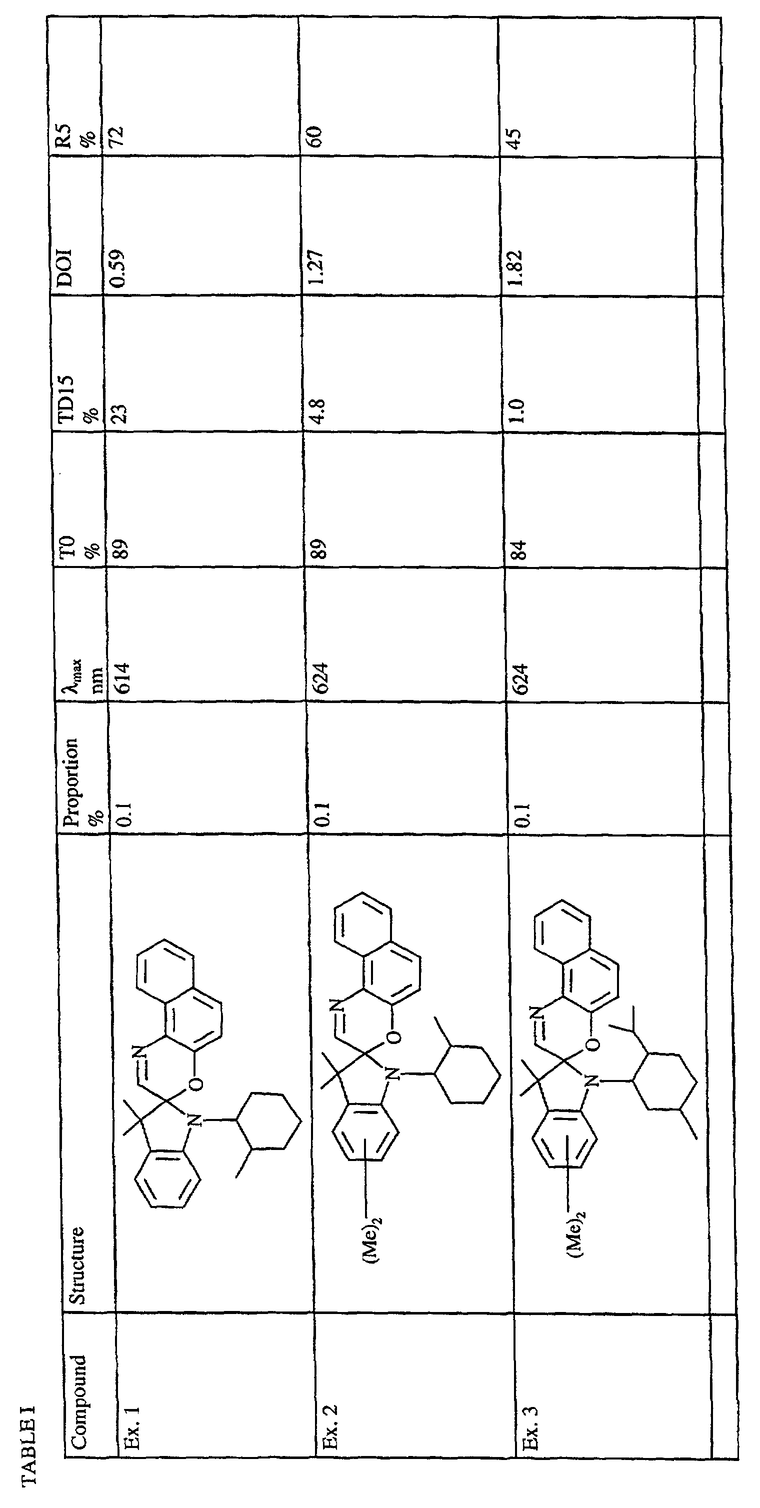

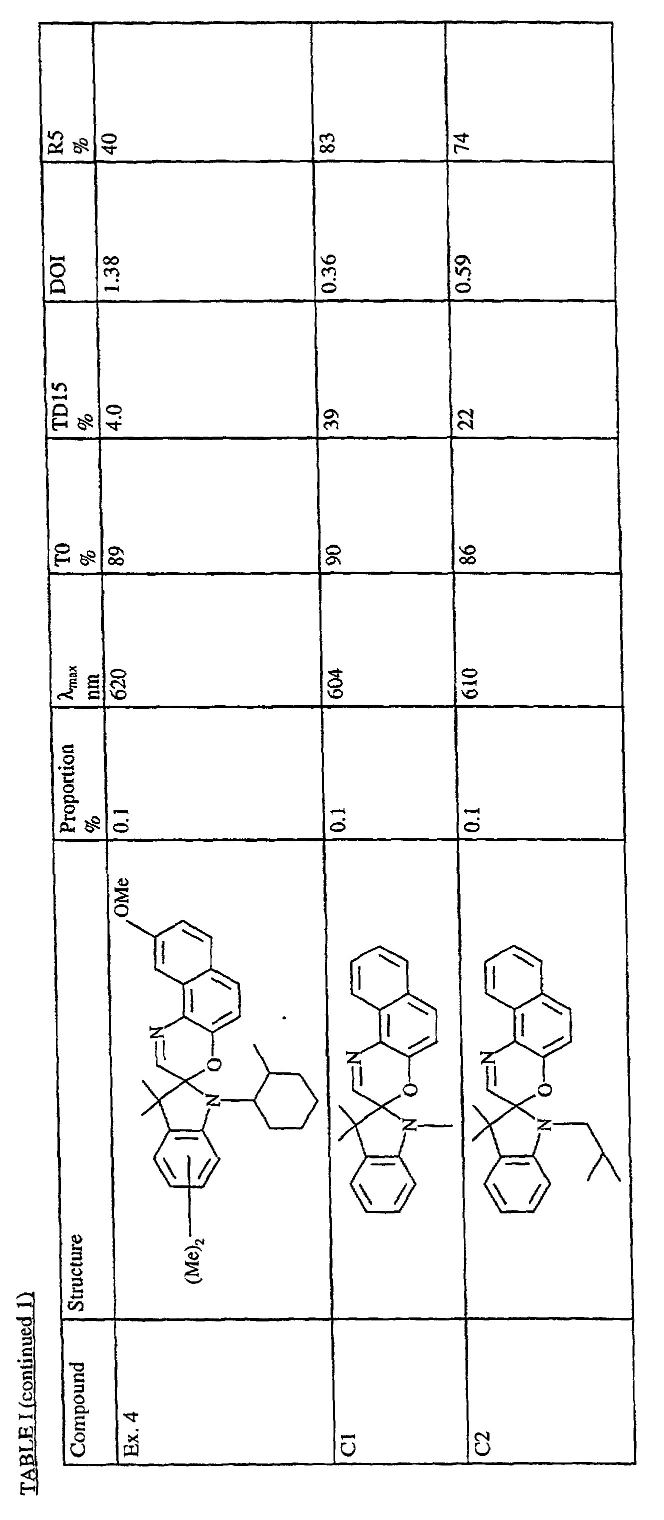

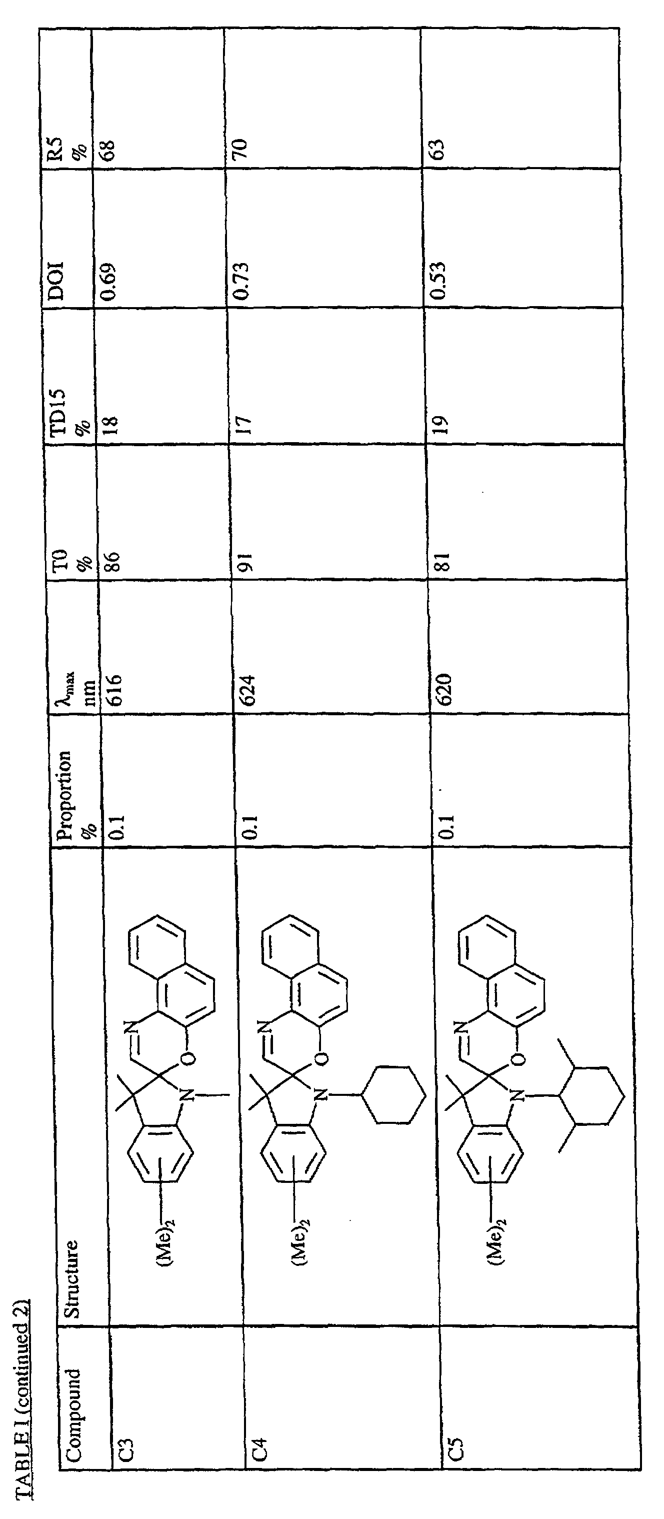

A comparison of the properties of Example 1 and Comparative Examples C1 and C2, on the one hand, and Examples 2 and 3 and Comparative Examples C3-C5, on the other hand, shows that the compounds of the prior art with analogous structure but without an asymmetric aliphatic monocyclic group according to the invention, do not possess the advantageous combination of the wanted properties. In particular, it is observed that the compounds of the invention have a better compromise between low initial coloration and strong induced optical density.

## Claims

1. Photochromic compounds having the following general formula (I): 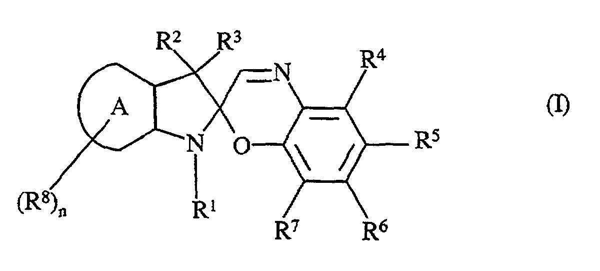 in which,
- R¹ is an asymmetric aliphatic monocyclic group which is directly connected to the nitrogen atom, having from 5 to 7 members, and possibly comprising at least one heteroatom, and having the formula 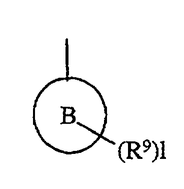 where 1 = 1-12, R⁹ represents H or an alkyl, alkoxy, dialkylamino, alkyl ester or CN group, provided that at least one of the R⁹ is not H,
- R², R³ are identical or different and they represent an alkyl group, linear or branched, of 1-12 carbon atoms, an alkenyl group, alkynyl, aryl, alkylaryl, cycloalkyl, R² and R³ can, optionally, combine to form a carbocyclic or heterocyclic group having 5 to 10 atoms,
- R⁴, R⁵, R⁶ and R⁷ are identical or different and they represent:
■ hydrogen,
■ an alkyl, cycloalkyl, alkenyl, alkynyl, aryl, heteroaryl, aryloxy or aralkyl, said group being optionally halogenated,
■ a halogen,
■ OR, SR, -OCOR, or -COOR, with R = H, alkyl and/or cycloalkyl and/or aryl,
■ a (poly)ether, a (poly)amide, a (poly)carbonate, a (poly)carbarnate, a (poly)urea or a (poly)ester,
■ an amino radical which gives rise, once it is bound in (I), to a primary, secondary or tertiary amine, said amine being alkyl, aryl or aralkyl, mono- or disubstituted depending on its nature,
or an aminocyclic radical containing one or more heteroatoms,
■ or an electron withdrawing group selected, preferably, from the group comprising CF₃, CN, NO₂ and SCN,
- where at least two of the radicals R⁴, R⁵, R⁶, R⁷, preferably borne by two adjacent carbon atoms, can combine to form at least one aromatic ring having 5 or 6 members or aliphatic ring having 5 to 7 members, preferably 5 or 6 members, said ring(s) comprising, optionally, of at least one heteroatom, so as to form at least one heterocyclic ring, the latter being optionally substituted by one or more radicals, which may be identical or different, and have the same definition as given above for R⁴ to R⁷,
- A represents a (hetero)aromatic ring, which is possibly substituted by one or more radicals R⁸, which may be identical or different and have the same definition as given above for R⁴ to R⁷, except hydrogen,
- n is a whole number and when n ≥ 2, two of the radicals R⁸ can possibly combine to form at least one aromatic or heteroaromatic ring.

2. Photochromic compounds according to claim 1, **characterized in that** group R¹ is selected from the following:

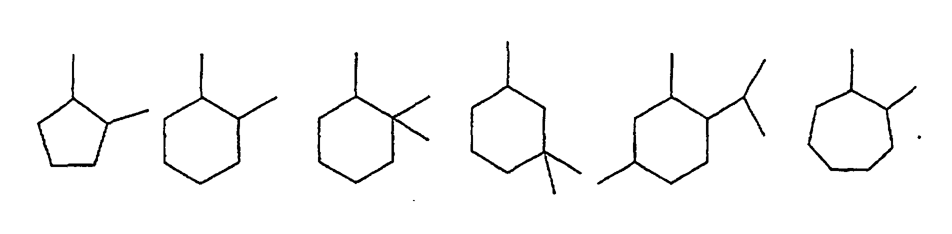

3. Photochromic compounds according to claim 1 or 2, **characterized in that** they have the general formula 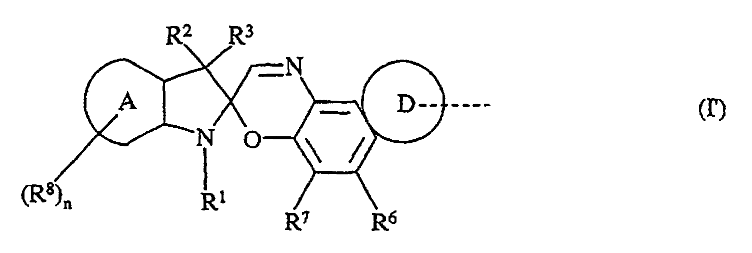 where A, R¹-R³, R⁶-R⁸ and n are as defined in the preceding claims, and D is an aromatic or aliphatic ring with 5 to 7 members, optionally comprising a heteroatom, which may or may not be substituted by one or more, identical or different, radicals, having the same definition as given for R⁴-R⁷ in the preceding claims, except hydrogen.

4. Photochromic compounds according to claim 1 or 2, **characterized in that** they have the following general formula (I"): 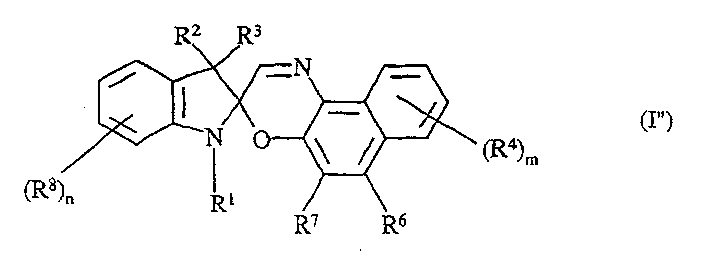 in which R¹-R⁴ and R⁶-R⁸ are as defined in claim 1, and n takes on values from 0 to 4 while m takes on values from 1 to 4

5. Photochromic compounds according to any one of claims 1 to 4, **characterized in that** the groups R¹-R⁹ of the Formulas (I), (I') and (I") according to the invention comprise and/or form at least one reactive polymerization and/or crosslinking group, selected from the following: alkenyl, methacryloyl, acryloyl, acryloxyalkyl, methacryloxyalkyl and epoxy.

6. Photochromic compounds according to any one of claims 1 to 5, **characterized in that** they present the following formula: 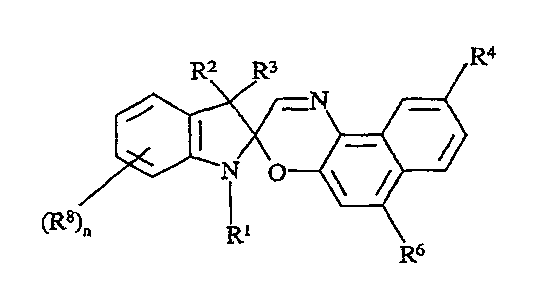 where R¹ = 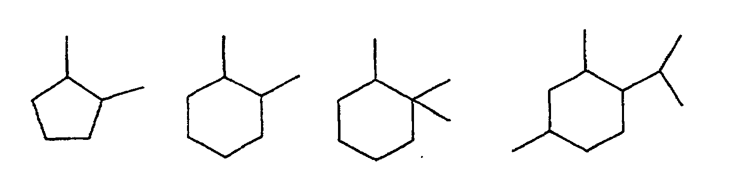
n = 0, 1 or 2, and
R², R³ = C₁-C₅ alkyl,
R⁴ = H, OMe,
R⁶ =H, OMe or amino,
R⁸ = Me, OMe or CF₃.

7. (Co)polymer and/or crosslinkage obtained by polymerization and/or crosslinking of at least one monomer consisting of at least one photochromic compound according to claim 5.

8. (Co)polymer, **characterized in that** it is grafted by means of at least one of the photochromic compounds according to any one of claims 1 to 6.

9. Photochromic composition, **characterized in that** it comprises:
- at least one compound according to any one of claims 1 to 6 and/or at least (co)polymer according to claim 7 or 8,
- and optionally at least one other photochromic compound and/or at least one dye and/or at least one stabilizer.

10. (Co)polymer matrix, **characterized in that** it comprises:
- at least one compound according to any one of claims 1 to 6,
- and/or at least one (co)polymer according to claim 7 or 8,
- and/or at least one composition according to claim 9.

11. Matrix according to claim 10, **characterized in that** the (co)polymer is selected from the following list:
- polyacrylate or polymethacrylate of alkyl, of cycloalkyl, of aryl or of arylalkyl (mono, di, tri or tetra) optionally halogenated or comprising at least one ether and/or ester and/or carbonate and/or carbamate and/or thiocarbamate and/or urea and/or amide group,
- polystyrene, polycarbonate, polyepoxy, polyurethane, polythiourethane, polysiloxane, polyacrylonitrile, polyamide, aliphatic or aromatic polyester, polyvinyl acetate, cellulose acetate, cellulose triacetate, cellulose acetate propionate or polyvinylbutyral,
- copolymers of two or more types of monomer or mixtures of the above-mentioned polymers.

12. Ophthalmic or sun-protection device comprising:
- at least one compound according to any one of claims 1 to 6,
- and/or at least one (co)polymer according to claim 7 or 8,
- and/or at least one composition according to claim 9.

13. Article according to claim 12, **characterized in that** it consists of a lens.

14. Glass partition and/or optical device comprising:
- at least one compound according to any one of claims 1 to 6,
- and/or at least one (co)polymer according to claim 7 or 8,
- and/or at least one composition according to claim 9,
- and/or at least one matrix according to claim 10 or 11.

## Patentansprüche

1. Photochrome Verbindungen der folgenden allgemeinen Formel (I) 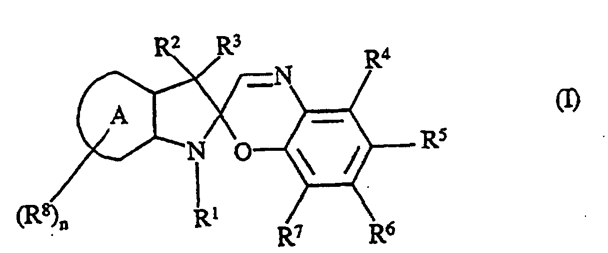 worin bedeuten:
- R¹ eine asymmetrische aliphatische monocyclische Gruppe, die mit dem Stickstoffatom direkt verbunden ist, 5 bis 7 Glieder aufweist und möglicherweise mindestens ein Heteroatom aufweist und die Formel hat 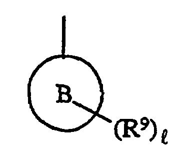 worin ℓ = 1-12, R⁹ steht für H oder eine Alkyl-, Alkoxy-, Dialkylamino-, Alkylester- oder CN-Gruppe, mit der Maßgabe, dass mindestens einer der Rest R⁹ nicht H bedeutet,
- R², R³ gleich oder verschieden sind und stehen für eine lineare oder verzweigte C₁-C₁₂-Alkylgruppe,
- Alkenylgruppe, -Alkinylgruppe, -Arylgruppe, -Alkylarylgruppe, -Cycloalkylgruppe, wobei R² und R³ gegebenenfalls miteinander kombiniert sein können unter Bildung einer carbocyclischen oder heterocyclischen Gruppe mit 5 bis 10 Atomen,
- R⁴, R⁵, R⁶ und R⁷ gleich oder verschieden sind und stehen für:
■ Wasserstoff,
■ eine Alkyl-, Cycloalkyl-, Alkenyl-, Alkinyl-, Aryl-, Heteroaryl-, Aryloxy- oder Aralkylgruppe, wobei die genannte Gruppe gegebenenfalls halogeniert sein kann,
■ ein Halogen,
■ OR, SR, -OCOR oder -COOR, worin R = H, Alkyl und/oder Cycloalkyl und/oder Aryl,
■ einen (Poly)Ether, ein (Poly)Amid, ein (Poly)Carbonat, ein (Poly)Carbamat, einen (Poly)Hamstoff oder einen (Poly)Ester,
■ einen Aminorest, der, wenn er einmal an (I) gebunden ist, ein primäres, sekundäres oder tertiäras Amin ergibt, das je nach seiner Art durch Alkyl, Aryl oder Aralkyl mono- oder disbstituiert ist,
oder einen aminocyclischen Rest, der ein oder mehr Heteroatome enthält,
■ oder eine Elektronen anziehende Gruppe, vorzugsweise ausgewählt aus der Gruppe, die CF₃, CN, NO₂ und SCN umfasst,
- wobei mindestens zwei der Reste R⁴, R⁵, R⁶, R⁷, die vorzugsweise von zwei benachbarten Kohlenstoffatomen getragen werden, miteinander kombiniert sein können unter Bildung mindestens eines aromatischen Ringes mit 5 oder 6 Gliedern oder eines aliphatischen Ringes mit 5 bis 7 Gliedern, vorzugsweise 5 oder 6 Gliedern, wobei der (die) genannte(n) Ring(e) gegebenenfalls mindestens ein Heteroatom umfasst (umfassen) unter Bildung mindestens eines heterocyclischen Ringes, der gegebenenfalls substituiert ist durch einen oder mehr gleiche oder verschiedene Reste, welche die gleiche Definition haben, wie sie oben für R⁴ bis R⁷ angegeben ist,
- A einen (hetero)aromatischen Ring, der möglicherweise durch einen oder mehr Reste R⁸ substituiert ist, die gleich oder verschieden sein können und die gleiche Definition haben, wie sie oben für R⁴ bis R⁷ angegeben ist, mit Ausnahme von Wasserstoff,
- n eine ganze Zahl und wobei dann, wenn n ≥ 2, zwei der Reste R⁸ möglicherweise kombiniert sein können unter Bildung mindestens eines aromatischen oder heteroaromatischen Ringes.

2. Photochrome Verbindungen nach Anspruch 1, **dadurch gekennzeichnet, dass** die Gruppe R¹ ausgewählt ist aus den folgenden :

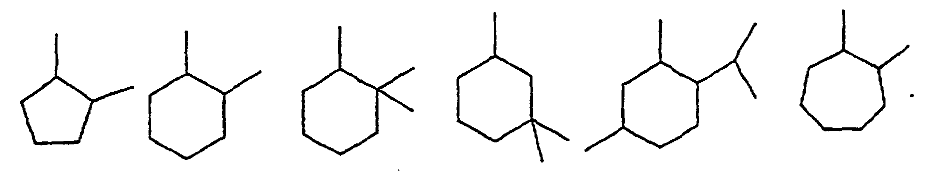

3. Photochrome Verbindungen nach Anspruch 1 oder 2, **dadurch gekennzeichnet, dass** sie die allgemeine Formel haben 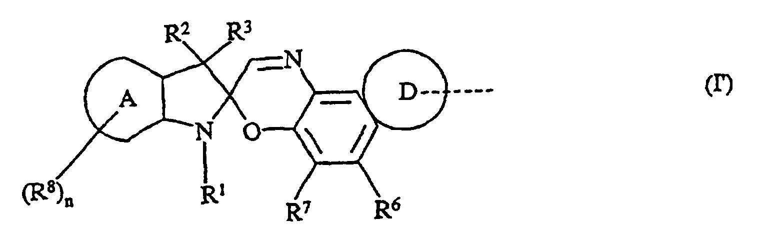 worin A, R¹ bis R³, R⁶ bis R⁸ und n wie in den vorhergehenden Ansprüchen definiert sind und D ein aromatischer oder aliphatischer Ring mit 5 bis 7 Gliedern ist, der gegebenenfalls ein Heteroatom umfasst und der unsubstituiert sein kann oder substituiert sein kann durch einen oder mehr gleiche oder verschiedene Reste, welche die gleiche Definition haben, wie sie in den vorhergehenden Ansprüchen für R⁴ bis R⁷ angegeben ist, mit Ausnahme von Wasserstoff.

4. Photochrome Verbindungen nach Anspruch 1 oder 2, **dadurch gekennzeichnet, dass** sie die folgende allgemeine Formel (I") haben: 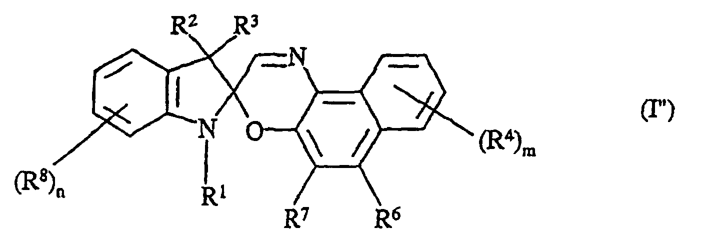 worin R¹ bis R⁴ und R⁶ bis R⁸ wie in Anspruch 1 definiert sind und n Werte von 0 bis 4 hat, während m Werte von 1 bis 4 hat.

5. Photochrome Verbindungen nach einem der Ansprüche 1 bis 4, **dadurch gekennzeichnet, dass** die Gruppen R¹ bis R⁹ der erfindungsgemäßen Formeln (I), (I') und (I") umfassen und/oder bilden mindestens eine reaktionsfähige Polymerisationsgruppe und/oder vernetzende Gruppe, ausgewählt aus den folgenden: Alkenyl, Methacryloyl, Acryloyl, Acryloxyalkyl, Methacryloxyalkyl und Epoxy.

6. Photochrome Verbindungen nach einem der Ansprüche 1 bis 5, **dadurch gekennzeichnet, dass** sie die folgende Formel haben: 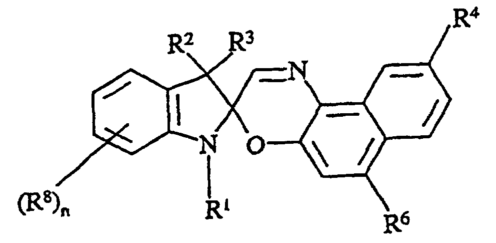 worin R¹ = 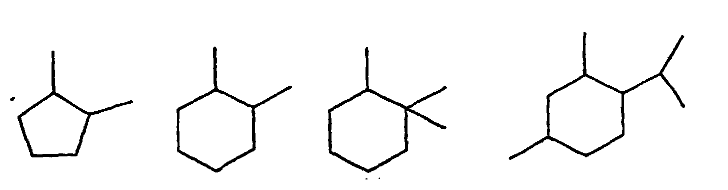
n = 0, 1 oder 2, und
R², R³ = C₁-C₅-Alkyl,
R⁴ = H, OMe,
R⁶ = H, OMe oder Amino,
R⁸ = Me, OMe oder CF₃.

7. (Co)Polymer und/oder Vernetzungsprodukt, erhalten durch Polymerisation und/oder Vernetzung mindestens eines Monomers, das besteht aus mindestens einer photochromen Verbindung nach Anspruch 5.

8. (Co)Polymer, **dadurch gekennzeichnet, dass** es bepfropft ist mit mindestens einer der photochromen Verbindungen nach einem der Ansprüche 1 bis 6.

9. Photochrome Zusammensetzung, **dadurch gekennzeichnet, dass** sie umfasst:
- mindestens eine Verbindung nach einem der Ansprüche 1 bis 6 und/oder mindestens ein (Co)Polymer nach Anspruch 7 oder 8,
- und gegebenenfalls mindestens eine andere (weitere) photochrome Verbindung und/oder mindestens einen Farbstoff und/oder mindestens einen Stabilisator.

10. (Co)Polymer-Matrix, **dadurch gekennzeichnet, dass** sie umfasst:
- mindestens eine Verbindung nach einem der Ansprüche 1 bis 6,
- und/oder mindestens ein (Co)Polymer nach Anspruch 7 oder 8,
- und/oder mindestens eine Zusammensetzung nach Anspruch 9.

11. Matrix nach Anspruch 10, **dadurch gekennzeichnet, dass** das (Co)Polymer ausgewählt ist aus der folgenden Liste:
- Alkyl-, Cycloalkyl-, Aryl- oder Arylalkyl- (mono, di, tri oder tetra)polyacrylat oder -polymethacrylat, das gegebenenfalls halogeniert ist oder mindestens eine Ether- und/oder Ester- und/oder Carbonat- und/oder Carbamat- und/oder Thiocarbamat- und/oder Harnstoff- und/oder Amidgruppe umfasst,
- Polystyrol, Polycarbonat, Polyepoxy, Polyurethan, Polythiourethan, Polysiloxan, Polyacrylnitril, Polyamid, aliphatischer oder aromatischer Polyester, Polyvinylacetat, Celluloseacetat, Cellulosetriacetat, Celluloseacetatpropionat oder Polyvinylbutyral,
- Copolymere von zwei oder mehr Typen von Monomeren oder Mischungen der oben genannten Polymeren.

12. Ophthalmische oder Sonnenschutz-Einrichtung, die umfasst:
- mindestens eine Verbindung nach einem der Ansprüche 1 bis 6,
- und/oder mindestens ein (Co)Polymer nach Anspruch 7 oder 8,
- und/oder mindestens eine Zusammensetzung nach Anspruch 9.

13. Artikel bzw. Gegenstand nach Anspruch 12, **dadurch gekennzeichnet, dass** er aus einer Linse besteht.

14. Glastrennwand und/oder optische Einrichtung, die umfasst (umfassen):
- mindestens eine Verbindung nach einem der Ansprüche 1 bis 6,
- und/oder mindestens ein (Co)Polymer nach Anspruch 7 oder 8,
- und/oder mindestens eine Zusammensetzung nach Anspruch 9,
- und/oder mindestens eine Matrix nach Anspruch 10 oder 11.

## Revendications

1. Composés photochromiques de formule générale (I) suivante : 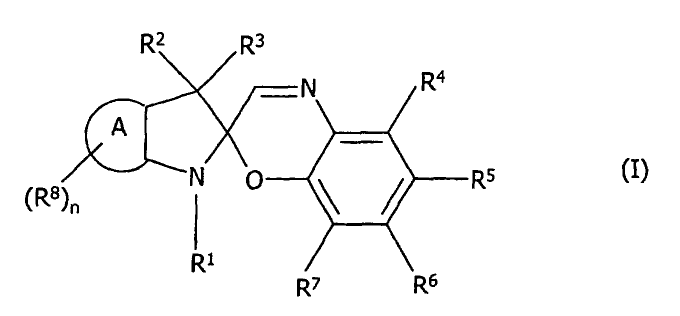 dans laquelle,
- R¹ est un groupe monocyclique aliphatique dissymétrique directement lié à l'atome d'azote, ayant de 5 à 7 chaînons, pouvant comporter au moins un hétéroatome et répondant à la formule 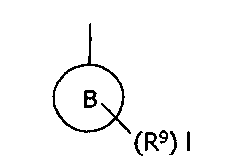 où I = 1 à 12, et R⁹ est H ou un groupe alkyle, alcoxy, dialkylamino, alkylester ou CN avec la condition qu'au moins l'un des R⁹ n'est pas H,
- R², R³ sont identiques ou différents et représentent un groupement alkyle, linéaire ou ramifié, de 1 à 12 atomes de carbone, alcényle, alcynyle, aryle, alkylaryle, cycloalkyle, R² et R³ pouvant, éventuellement, être pris ensemble pour former un groupement carbocyclique ou hétérocyclique de 5 à 10 atomes,
- R⁴, R⁵, R⁶ et R⁷ sont identiques ou différents et représentent :
■ l'hydrogène
■ un groupement alkyle, cycloalkyle, alcényle, alcynyle, aryle, hétéroaryle, aryloxy ou aralkyle, ledit groupement étant éventuellement halogéné,
■ un halogène,
■ OR, SR, -OCOR ou -COOR, avec R = H, alkyle et/ou cycloalkyle et/ou aryle,
■ un (poly)éther, un (poly)amide, un (poly)carbonate, un (poly)carbamate, un (poly)urée ou un (poly)ester,
■ * un radical amino donnant naissance, une fois lié dans (I), à une amine primaire, secondaire ou tertiaire, ladite amine étant alkyl-, aryl- ou aralkyl-, mono- ou disubstituée selon sa nature,
* ou un radical aminocyclique contenant un ou plusieurs hétéroatomes,
■ ou un groupement électroattracteur choisi de préférence dans le groupe comprenant CF₃, CN, NO₂ et SCN,
- au moins deux des radicaux R⁴, R⁵, R⁶, R⁷, portés de préférence par deux atomes de carbone adjacents, pouvant se combiner pour former au moins un cycle aromatique ayant 5 ou 6 chaînons ou aliphatique ayant 5 à 7 chaînons, de préférence 5 ou 6 chaînons, ledit (ou lesdits) cycle(s) comprenant, éventuellement, au moins un hétéroatome, de façon à former au moins un noyau hétérocyclique, ce dernier étant éventuellement substitué par un ou plusieurs radicaux qui peuvent être identiques ou différents, et répondant à la même définition que celle donnée supra pour R⁴ à R⁷,
- A représente un cycle (hétéro)aromatique qui est éventuellement substitué par un ou plusieurs radicaux R⁸, qui peuvent être identiques ou différents, et répondent à la même définition que celle donnée supra pour R⁴ à R⁷, sauf l'hydrogène,
- n est un nombre entier et quand n ≥ 2, deux des radicaux R⁸ peuvent éventuellement être pris ensemble pour former au moins un cycle aromatique ou hétéroaromatique.

2. Composés photochromiques selon la revendication 1, **caractérisés en ce que** le groupe R¹ est choisi parmi les suivants :

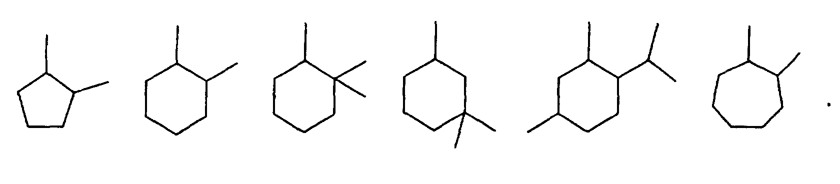

3. Composés photochromiques selon la revendication 1 ou 2, **caractérisés en ce qu'**ils répondent à la formule générale 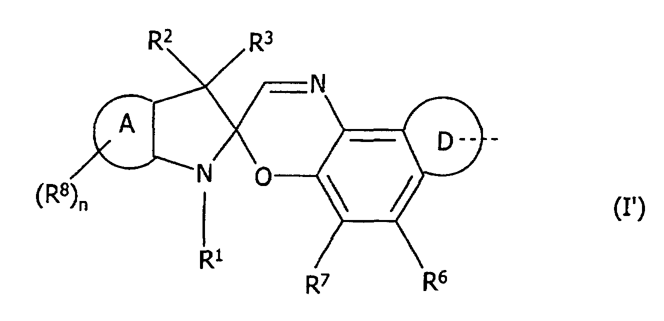 où A, R¹-R³, R⁶-R⁸ et n sont tels que définis dans les revendications précédentes, et D est un cycle aromatique ou aliphatique de 5 à 7 chaînons, comprenant éventuellement un hétéroatome, qui peut être substitué ou non par un ou plusieurs radicaux, identiques ou différents, répondant à la même définition que celle donnée pour R⁴ à R⁷ dans les revendications précédentes, sauf l'hydrogène.

4. Composés photochromiques selon la revendication 1 ou 2, **caractérisés en ce qu'**ils ont la formule générale (I") suivante : 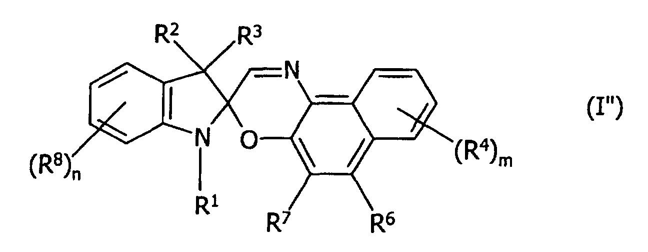 dans laquelle R¹ à R⁴ et R⁶ à R⁸ sont tels que définis dans la revendication 1, et n prend des valeurs de 0 à 4 tandis que m prend des valeurs de 1 à 4.

5. Composés photochromiques selon l'une quelconque des revendications 1 à 4, **caractérisés en ce que** les groupements R¹ à R⁹ des formules (I), (I') et (I") selon l'invention, comprennent et/ou forment au moins un groupe réactif de polymérisation et/ou réticulation, sélectionné parmi les suivants : alcényle, méthacryloyle, acryloyle, acryloxyalkyle, méthacryloxyalkyle et époxy.

6. Composés photochromiques selon l'une quelconque des revendications 1 à 5, **caractérisés en ce qu'**ils présentent la formule suivante: 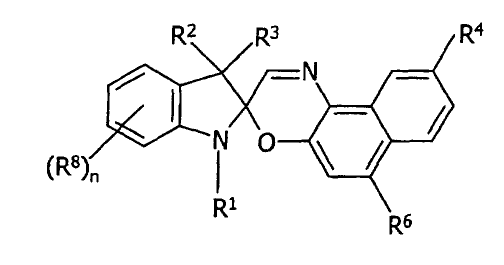 où R¹ = 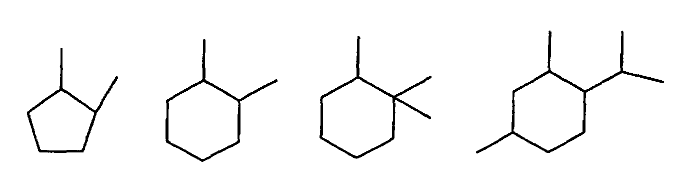
n = 0, 1 ou 2 et
R², R³ = alkyle en C₁-C₅,
R⁴ = H, OMe,
R⁶ = H, OMe ou amino,
R⁸ = Me, OMe ou CF₃.

7. (Co)polymère et/ou réticulat obtenu par polymérisation et/ou réticulation d'au moins un monomère constitué par au moins un composé photochromique selon la revendication 5.

8. (Co)polymère, **caractérisé en ce qu'**il est greffé à l'aide d'au moins l'un des composés photochromiques selon l'une quelconque des revendications 1 à 6.

9. Composition photochromique, **caractérisée en ce qu'**elle comprend :
- au moins un composé selon l'une quelconque des revendications 1 à 6 et/ou au moins un (co)polymère selon la revendication 7 ou 8,
- et éventuellement au moins un autre composé photochromique et/ou au moins un colorant et/ou au moins un stabilisant.

10. Matrice (co)polymère, **caractérisée en ce qu'**elle comprend :
- au moins un composé selon l'une quelconque des revendications 1 à 6,
- et/ou au moins un (co)polymère selon la revendication 7 ou 8,
- et/ou au moins une composition selon la revendication 9.

11. Matrice selon la revendication 10, **caractérisée en ce que** le (co)polymère est choisi dans la liste suivante :
- polyacrylate ou polyméthacrylate d'alkyle, de cycloalkyle, d'aryle ou d'arylalkyle (mono, di, tri ou tétra) éventuellement halogéné ou comportant au moins un groupement éther et/ou ester et/ou carbonate et/ou carbamate et/ou thiocarbamate et/ou urée et/ou amide,
- polystyrène, polycarbonate, polyépoxy, polyuréthane, polythiouréthane, polysiloxane, polyacrylonitrile, polyamide, polyester aliphatique ou aromatique, acétate de polyvinyle, acétate de cellulose, triacétate de cellulose, acétate-propionate de cellulose ou polyvinylbutyral,
- copolymères de deux ou plusieurs types de monomères ou mélanges des polymères visés supra.

12. Dispositif ophtalmique ou solaire comprenant :
- au moins un composé selon l'une quelconque des revendications 1 à 6,
- et/ou au moins un (co)polymère selon la revendication 7 ou 8,
- et/ou au moins une composition selon la revendication 9.

13. Article selon la revendication 12, **caractérisé en ce qu'**il est constitué par une lentille.

14. Vitrage et/ou dispositif optique comprenant :
- au moins un composé selon l'une quelconque des revendications 1 à 6,
- et/ou au moins un (co)polymère selon la revendication 7 ou 8,
- et/ou au moins une composition selon la revendication 9,
- et/ou au moins une matrice selon la revendication 10 ou 11.
